# EUROPEAN PATENT SPECIFICATION

(11) **EP 2 411 008 B1**
(45) Date of publication and mention of the grant of the patent: **22.03.2017**
(21) Application number: 10717888.1
(22) Date of filing: 26.03.2010
(51) Int. Cl.: A61K 31/46, A61K 47/61, A61P 11/06, A61K 45/06, A61K 9/00

(54) **COMPLEXES OF TIOTROPIUM WITH IMPROVED PROPERTIES OF SOLUBILITY AND STABILITY**
TIOTROPIUM-KOMPLEXE MIT VERBESSERTEN LÖSLICHKEIT- UND STABILITÄTSEIGENSCHAFTEN
COMPLEXES DE TIOTROPIUM CHARACTERISEES DUNE SOLUTION ET STABILISATION AMELIOREE

(30) Priority: 27.03.2009 TR 200902394
(43) Date of publication of application: 01.02.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000056
(87) International publication number: WO 2010/110760

(56) References cited:
- EP-A1- 1 894 559
- WO-A1-03/066031
- WO-A1-2005/042528
- DE-A1- 10 126 924
- LEITE PINTO JMC ET AL: "Beclomethasone/cyclodextrin inclusion complex for dry powder inhalation" S.T.P. PHARMA SCIENCES, vol. 9, no. 3, May 1999 (1999-05), pages 253-256, XP9134469 ISSN: 1157-1489

## Description

### Field of the invention

Present invention relates to complexes for improving solubility and stability properties of an active agent, pharmaceutical composition comprising these complexes, preparation processes of these compositions and use of these compositions for the treatment of respiratory disorders.

### Background of the invention

Present invention relates to pharmaceutical composition suitable for delivery via inhalation, comprising tiotropium or a pharmaceutically acceptable salt, ester, solvate, derivative, polymorph or hydrate and having improved properties of solubility and stability.

Tiotropium (Formula I), is an anticholinergic agent with a chemical name (1α, 2β, 4β, 5α, 7β)-7-[(hydroxidi-2-thienylacetyl)oxy]-9,9-dimethyl-3-oxa-9-azoniatricyclo [3.3.1.0^{2,4}] nonane.

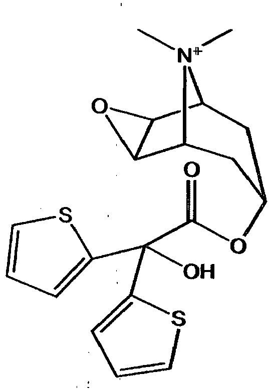

Tiotropium, is described in patent application EP0418716 (A1) (USRE39820 E1, US5610163 A and WO9104252 A1 among its patent family) for the first time. The application relates to processes for preparing tiotropium, pharmaceutical compositions containing tiotropium, long-acting, strong anticholinergic activity of tiotropium and use of it in the treatment of respiratory disorders.

Tiotropium is a long acting, strong anticholinergic bronchodilator, which is administered orally as dry powder inhalation for the treatment of respiratory disorders. Tiotropium antagonizes the effect of acetylcholine by blocking cholinergic muscarinic receptors. Tiotropium is separated slowly from M1 and M3 receptors causing broncho-construction, but separated rapidly from M2 receptors inhibiting release of acetylcholine from cholinergic nerve endings. This situation occured in lung receptors demonstrates long acting bronchodilator activity of the drug.

Inhalation route is a commonly preferred treatment method for respiratory disorders especially chronic disorders such as asthma and chronic obstructive pulmonary disorder (COPD) which become threatening widely in the society. The reason is that drug reaches directly and rapidly to target area; by comparison with doses required to be administered via oral or parenteral route, lower doses show desired effect depending on delivering the drug to target area directly and drug which is used in lower doses show less side-effect. Gastrointestinal disturbances, such as low resolution, low permeability, drug irritation, production of undesired metabolites and decrease of bioavailability depending on food, are felt in minimum level because drugs administered via inhalation route are not exposed to gastrointestinal medium.

In addition to advantages of inhalation treatment, there are various difficulties that should be considered during formulating active ingredient suitable for use by inhalation. For example, to provide optimum effectiveness, particles included in said composition should meet essential requirements like high solubility, suitable particle size, uniformity of particle size distribution and high physical and chemical stability.

"Stability" generally means that stability displayed by the active ingredient both in environmental conditions, and during production of the formulation and also after converted into the final product. The reason of why concept of stability have come into prominence, is the importance of that depending on administering low dose the amount of active agent delivered to target area have not to be less than required amount.

Another factor efficient for providing optimum effectiveness is property of solubility. Method used widely in optimizing solubility, is the decrease of particle size. However, since particles having only a certain size (1-10µm, preferably <5µm) can be transferred to the lungs via inhalation, it is not possible to change particle size to improve solubility properties. Because, on the other hand, drug particles should be constant in certain particle size for reaching to target area as desired and for being absorbed there; in other words, homogenization or uniformity of particle size distribution should be provided. Besides, due to affecting rate of drug release and absorption of drug, consequently effectiveness; solubility is a factor which should be kept under control. For inhaled drug particles, absorption rate in the area of absorption is a function of solubility. Drugs with low solubility are cleaned with ciliary activity before being absorbed in target area. Drugs with high solubility are easily dissolved in mucosal liquids and exhibit a rapid absorption in respiratory tract's tissues. In this case, formulation comprising active agent should have a high solubility.

In conclusion, formulations which can meet said requirements are needed. Various documents in the prior art about improving solubility and stability properties of pharmaceutical formulations of tiotropium suitable for the delivery via inhalation.

In the patent application US20080057129 A1, pharmaceutical compositions comprising a micronized carrier carrying micronized drug microparticles have been described. In the application, it is defined that when pharmaceutical compositions comprising a low soluble active agent selected from a group including also tiotropium are delivered via inhalation, they dissolve adequately in lungs.

In the patent application WO2006066367 A1, pharmaceutical compositions are formed by solid particles comprising at least one lipidic compound and phospholipids distributed homogenously, have been described. In the application, it is defined that said pharmaceutical compositions comprising high amount phospholipid and a low soluble active agent selected from the group including also tiotropium improve drug release and absorption.

In the patent application WO2007068443 A1, powder compositions suitable for inhalation comprising fine particles of a drug and fine particles of a force-controlling agent wherein the particles of said force-controlling agent are disposed on the surface of the active particles, are described. In the application, it is defined that pharmaceutical compositions are stable, comprising particles of an active agent selected from the group including also tiotropium and particles of a force-controlling agent selected from aminoacids, peptids, polypeptids, phospholipids, titanium dioxide, aluminum dioxide, silicon dioxide, starch and salts of fatty acids.

In the patent application WO2003084509 A1, a method for preparing a physically stable and homogenous powdered compositions containing tiotropium in admixture with a physiologically acceptable excipient, is described. In the application, it is defined that powdered preparations are stable, obtained by a method wherein tiotropium salt and excipient are formed a suspension with a suspension agent in which tiotropium salt and excipient are insoluble, and afterwards suspension agent is removed.

DE10126924 A1 discloses inhalation capsules filled with an inhalable powdered mixture of tiotropium (I) and an auxiliary (II), the capsule material (III) has a reduced moisture content, i.e. a Tews or halogen drier moisture content of less than 15 %.

In conclusion, documents are limited which submit solution suggestions with regard to improving solubility and stability properties of pharmaceutical compositions of tiotopium suitable for delivery via inhalation. Besides, these documents doesn't exhibit a proposal directed to improving at the same time solubility and stability properties of tiotropium. For this reason, new tiotropium formulations comprising improved solubility and stability properties, which are suitable for the transference via inhalation, are needed.

### Summary of the invention

Present invention relates to pharmaceutical composition in dry powder form suitable for the delivery via inhalation, is characterized by containing a complex of tiotropium or a pharmaceutically acceptable salt, ester, solvate, polymorph or hydrate thereof and β-cyclodextrin or derivatives thereof, said complex having an average particle size ranging from 1 to 10 µm, wherein the β-cyclodextrin derivatives are 2-hydroxypropyl-β-cyclodextrin, sulfobutylether-β-cyclodextrin, the sodium salt of β-cyclodextrin, randomly methylated β-cyclodextrin or branched β-cyclodextrin.

### Detailed Description of the Invention

Present invention submits pharmaceutical compositions suitable for the delivery via inhalation, comprising a non-toxic, pharmaceutically acceptable and therapeutically effective amount of tiotropium or a pharmaceutically acceptable salt, solvate, polymorph or hydrate thereof and having improved solubility and stability properties.

According to the invention, complex of tiotropium or pharmaceutically acceptable salt, ester, solvate, polymorph or hydrate thereof as active ingredient, with cyclodextrin or derivatives thereof has improved solubility and stability properties of pharmaceutical compositions. As defined above, solubility is closely related with rate of drug release and absorption of drug. By this means, since each dose taken is absorbed rapidly and effectively, desired activity has been reached by administering lower dose and consequently, drug administered with lower dose has showed less side effect. Besides, since said pharmaceutical compositions do not encounter any stability problem, depending on that administered dose is low, possibility of that the amount of active ingredient reaching to target area lower than the required amount, is removed. In conclusion, hence solubility and stability properties of pharmaceutical compositions of the invention comprising a complex of tiotropium or a pharmaceutically acceptable salt, ester, solvate, derivative, polymorph or hydrate thereof and cyclodextrin or derivatives thereof, are improved; said pharmaceutical compositions have provided a great improvment beyond meeting requirements of therapeutic effectiveness and also of security.

"Tiotropium or a pharmaceutically acceptable salt, ester, solvate, polymorph or hydrate thereof" means preferably tiotropium bromide.

β-cyclodextrin derivatives mean substances which can be 2-hydroxypropil-β-cyclodextrin, sulfobutylether β-cyclodextrin, the sodium salt of β-cyclodextrin, randomly methylated β-cyclodextrin and branched β-cyclodextrin.

Pharmaceutical compositions of the invention are in the form of dry powder inhalation composition or pressurized metered dose inhalation composition.

During the preparation of dry powder inhalation composition, there are two methods which are widely applied for delivery of drug in required amount to target area. One of them is the controlled agglomeration of non-diluted drug; the other is based on the adhesion of micronized drug particles to surface of inert carrier of large particle size. Pharmaceutical compositions in accordance with the invention are prepared by using preferably the second method. When the second method is used, dry powder inhalation compositions in accordance with the invention containing optionally at least one pharmaceutically acceptable inert carrier and at least one other pharmaceutically acceptable excipient besides complex of tiotropium bromide/β-cyclodextrin.

The term "micronized drug particles" means particles of tiotropium bromide/β-cyclodextrin complex. Tiotropium bromide/β-cyclodextrin complex is characterized by having average particle size ranging from 1 to 10 µm, preferably ranging from 1 to 5µm. Pharmaceutical compositions in accordance with the invention are characterized by comprising tiotropium bromide/β-cyclodextrin complex in a range of 0.001 % to 50%, preferably 0.01 % to 10% by weight.

The term "inert carrier" means a carrier which is preferably lactose for dry powder inhalation composition in accordance with the invention. Pharmaceutical compositions in accordance with the invention can contain at least one inert carrier of large particle size and of small particle size, and optionally at least one excipient. Inert carrier of large particle size in accordance with the invention is characterized by having an average particle size (d₅₀) ranging from 10 to 250 µm, preferably from 10 to 150 µm, more preferably 150 µm; inert carrier of small particle size is characterized by having an average particle size (d₅₀) ranging from 1 to 10 µm, preferably 10 µm. As carriers of large particle and of small particle, which have different particle size, can be same, they can also be different.

At least one pharmaceutically acceptable excipient can be selected from the group consisting of carbohydrates such as lactose, glucose, fructose, galactose, sucrose, maltose, trehalose, maltodextrins, dextrans, starch and cellulose; polyalcohols such as sorbitol, mannitol and xylitol; amino acids such as glycine, arginine, lysine, aspartic acid and glutamic acid; peptides such as human serum albumin; gelatin; various salts and taste masking agents. However, said pharmaceutically acceptable excipient is not limited with the ones disclosed above.

During the preparation of pressurized metered dose inhalation compositions, two formulation strategies are practiced depending on physico-chemical properties of the active ingredient and of the pressurized gas system. One of them is the solution formulation, the other is suspension formulation. Pharmaceutical composition in accordance with the invention contains propellants, surfactants and at least one essential excipient selected from the group of co-solvents and optionally at least one other pharmaceutically acceptable excipient, besides tiotropium bromide/β-cyclodextrin complex.

The term "active ingredient" means tiotropium bromide/β-cyclodextrin complex. Tiotropium bromide/β-cyclodextrin complex in accordance with the invention is characterized by having an average particle size ranging from 1µm to 10 µm, preferably from 1µm to 5µm. Presurrized metered dose inhalation compositions in accordance with the invention are characterized by containing tiotropium bromide/β-cyclodextrin complex in the proportion of 0.001% to 50%, preferably 0.01% to 10% by weight.

Depending on the formulation strategy, at least one pharmaceutically acceptable excipient can be selected from the group consisting of propellants such as chlorofluorocarbons, hydrofluoroalkanes and hydrocarbons; surfactants such as oleic acid, polysorbates, propylene glycole, polyethylene glycol, cetyl alcohol, stearyl alcohol, sorbitan fatty acid esters, sugar esters of fatty acid, glycerides of fatty acids, isopropyl myristate and lecithin; cosolvents such as ethanol, water and diethyl ether; antioxydants such as butylhydroxyanisol (BHA), sodium ascorbate, butylhydroxytoluen (BHT), sodium sulfite, gallates (such as propyl gallate), tocopherol, citric acid, malic acid, ascorbic acid, acetylcysteine, fumaric acid, lecithin, ascorbyl palmitate, ethylendiamine tetraacetate; and sweeteners. However, said pharmaceutically acceptable excipient is not limited with the ones disclosed above.

As described above, pharmaceutical compositions in accordance with the invention are in the form of dry powder inhalation composition or pressurized metered dose inhalation composition, preferably in the form of dry powder inhalation composition.

Pharmaceutical compositions containing tiotropium bromide/β-cyclodextrin complex in accordance with the invention, can further contain at least one active ingredient selected from the group consisting of other anticholinergic agents, adrenergic agonists, antiallergic agents, anti-inflammatory agents, antihistaminics, steroids, leukotriene receptor antagonists, antimuscarinic agents, PDE inhibitors and EGFR inhibitors. Tiotropium bromide/β-cyclodextrin complex and at least one active ingredient selected from defined group can be administered separately, sequentially and simultaneously.

Another aspect of the invention, pharmaceutical compositions containing tiotropium bromide/β-cyclodextrin complex in accordance with the invention are used in the treatment of respiratory disorders, especially asthma and COPD.

Tiotropium bromide/β-cyclodextrin complex in accordance with the invention can be prepared in solution or suspension or as solid by using conventional methods as precipitation, neutralization, slurry, agglomeration, grinding, freeze drying, spray drying according to the type of pharmaceutical composition.

Pharmaceutical compositions of dry powder inhalation in accordance with the invention can be prepared by a method comprising the following steps;
- sieving inert carrier with coarse particle size and fine particle size separately,
- obtaining first mixture by adding sieved inert carrier with fine particle size to tiotropium bromide/β-cyclodextrin complex,
- sieving obtained first mixture
- obtaining second mixture by adding sieved inert carrier with coarse particles to the first mixture,
- sieveing and mixing obtained second mixture
- making the final mixture ready for capsule filling.

Pharmaceutical compositions of pressurized metered dose inhalation in accordance with the invention can be prepared by a method comprising the following steps;
- cooling manufacture vessel to -25°C,
- pumping the propellant to the vessel
- adding tiotropium bromide/β-cyclodextrin complex to vessel and mixing the obtained mixture,
- filling the final mixture to suitable containers.

Examples of pharmaceutical formulations in accordance with the invention are listed below. These examples are given to explain the invention, and this invention is not limited by these examples.

### Example 1: Formulation of dry powder inhalation

| Content | Amount (mg) |
|---|---|
| Tiotropium bromide/β-cyclodextrin complex | 0.1125 |
| Lactose monohydrate (d₅₀=150µm) | 19.9500 |
| Lactose monohydrate (d₅₀=10µm) | 4.9375 |
| Total weight | 25 |

### Example 2: Formulation of dry powder inhalation

| Content | Amount (mg) |
|---|---|
| Tiotropium bromide/β-cyclodextrin complex | 0.1117 |
| Lactose monohydrate (d₅₀=150µm) | 19.9500 |
| Lactose monohydrate (d₅₀=10µm) | 4.9383 |
| Total weight | 25 |

### Example 3: Formulation of pressurized metered dose inhalation

| Content | Amount (mg) |
|---|---|
| Tiotropium bromide/β-cyclodextrin complex | 0.1125 |
| HFA 134 | 74.98 |
| Total weight | 75.0925 |

### Example 4: Formulation of pressurized metered dose inhalation

| Content | Amount (mg) |
|---|---|
| Tiotropium bromide/β-cyclodextrin complex | 0.1117 |
| HFA 227 | 74.98 |
| Total weight | 75.0925 |

## Claims

1. Pharmaceutical composition in dry powder form suitable for the delivery via inhalation, is **characterized by** containing a complex of tiotropium or a pharmaceutically acceptable salt, ester, solvate, polymorph or hydrate thereof and β-cyclodextrin or derivatives thereof, said complex having an average particle size ranging from 1 to 10 µm, wherein the β-cyclodextrin derivatives are 2-hydroxypropyl-β-cyclodextrin, sulfobutylether-β-cyclodextrin, the sodium salt of β-cyclodextrin, randomly methylated β-cyclodextrin or branched β-cyclodextrin.

2. Pharmaceutical composition according to Claim 1, is **characterized by** comprising preferably tiotropium bromide salt as tiotropium or a pharmaceutically acceptable salt, ester, solvate, polymorph or hydrate thereof.

3. Pharmaceutical composition according to Claim 1, is **characterized in that** complex is formed by tiotropium or a pharmaceutically acceptable salt, ester, solvate, polymorph or hydrate thereof and β-cyclodextrin or derivatives thereof, which have an average particle size ranging from 1 to 5µm, and wherein the β-cyclodextrin derivatives are 2-hydroxypropyl-β-cyclodextrin, sulfobutylether-β-cyclodextrin, the sodium salt of β-cyclodextrin, randomly methylated β-cyclodextrin or branched β-cyclodextrin.

4. Pharmaceutical composition according to Claim 1, is **characterized by** containing the complex formed by tiotropium or a pharmaceutically acceptable salt, ester, solvate, polymorph or hydrate thereof and β-cyclodextrin or derivatives thereof, in the proportion of 0.001% to 50%, wherein the β-cyclodextrin derivatives are 2-hydroxypropyl-β-cyclodextrin, sulfobutylether-β-cyclodextrin, the sodium salt of β-cyclodextrin, randomly methylated β-cyclodextrin or branched β-cyclodextrin.

5. Pharmaceutical composition according to Claim 1, is **characterized by** containing the complex formed by tiotropium or a pharmaceutically acceptable salt, ester, solvate, polymorph or hydrate thereof and β-cyclodextrin or derivatives thereof, in the proportion of 0.01% to 10% by weight, wherein the β-cyclodextrin derivatives are 2-hydroxypropyl-β-cyclodextrin, sulfobutylether-β-cyclodextrin, the sodium salt of β-cyclodextrin, randomly methylated β-cyclodextrin or branched β-cyclodextrin.

6. Pharmaceutical composition according to Claim 1, is **characterized by** further comprising at least one active ingredient selected from the group of other anticholinergic agents, adrenergic agonists, antiallergic agents, anti-inflammatory agents, antihistaminics, steroids, leukotriene receptor antagonists, antimuscarinic agents, PDE inhibitors and EGFR inhibitors for administering separately, sequentially and simultaneously.

7. Pharmaceutical composition according to any preceding claim, **characterized by** being in the form of dry powder inhalation composition or pressurized metered dose inhalation composition, preferably in the form of dry powder inhalation composition.

8. A Pharmaceutical composition according to claim 8 for use in a method of treating respiratory disorders, especially asthma and COPD.

## Patentansprüche

1. Pharmazeutische Zusammensetzung, welche geeignet für inhalation in Form eines trockenen Pulvers ist, **dadurch gekennzeichnet, dass** sie einen Komplex von Tiotropium oder ein pharmazeutisch verträgliches Salz, Ester, Solvat, polymorphe form oder Hydrats davon und β -Cyclodextrinderivate oder Derivate davon enthält, wobei dieser Komplex eine durchschnittlichen Partikelgrößen zwischen im Bereich von 1 bis 10µm aufweist, wobei die β -Cyclodextrin Derivaten 2-hydroxypropyl-β-Cyclodextrin, Sulfobutylether-β-Cyclodextrin, das Natriumsalz von β-Cyclodextrin, randommäßig methylierte β-Cyclodextrin oder verzweigte β-Cyclodextrin sind.

2. Pharmazeutische Zusammensetzung nach Anspruch 1, **dadurch gekennzeichnet, dass** welches aus Tiotropium, bevorzugt Tiotropiumbromid Salz oder ein pharmazeutisch verträgliches Salz, Ester, Solvat, polymorphe Form oder Hydrat zusammen gesetzt ist.

3. Pharmazeutische Zusammensetzung nach Anspruch 1, **dadurch gekennzeichnet, dass** welches aus einer Zusammensetzung von durchschnittlichen Partikelgrössen sind zwischen 1 und 5 µm Komplexe, und β-Cyclodextrinderivate 2-hydroxypropyl-β-Cyclodextrin Sulfobutylether- β-Cyclodextrin, β-Natriumsalz von Cyclodextrin, zufällige methylierte β-Cyclodextrin oder verzweigt β-Cyclodextrin ist, Tiotropium oder ein pharmazeutisch verträgliches Salz, Ester, Solvat, polymorphe Form oder Hydrat und aus β-Cyclodextrin besteht oder

4. Pharmazeutische Zusammensetzung nach Anspruch 1, **dadurch gekennzeichnet, dass** welches aus 0,001% bis 50% Tiotropium oder ein pharmazeutisch verträgliches Salz, Ester, Solvat, polymorphe Form oder Hydrat und β- aus dem Cyclodextrin oder Derivat davon gebildete 2-hydroxypropyl-β-Cyclodextrin Sulfobutylether-β-Cyclodextrin, β-Natriumsalz von Cyclodextrin, zufällige methylierte β-Cyclodextrin oder verzweigt β-Cyclodextrin Komplexe besteht.

5. Pharmazeutische Zusammensetzung nach Anspruch 1, **dadurch gekennzeichnet, dass** welches aus 0,01% bis 10% Tiotropium oder ein pharmazeutisch verträgliches Salz, Ester, Solvat, polymorphe Form oder Hydrat und β- aus dem Cyclodextrin oder Derivat davon gebildete, 2-hydroxypropyl-β-Cyclodextrin Sulfobutylether-β-Cyclodextrin, β-Natriumsalz von Cyclodextrin, zufällige methylierte β - Cyclodextrin oder verzweigt und aus Cyclodextrin Komplexe besteht.

6. Pharmazeutische Zusammensetzung nach Anspruch 1 **dadurch gekennzeichnet, dass** mit der einen gleichzeitig Verabreichung der anderen intermittierenden aktiven Anticholinergika, adrenerge Agonisten, Antiallergika, entzündungshemmende Mittel, Antihistaminika, Steroide, Leukotrien-Rezeptor-Antagonisten, Anticholinergika, PDE-Inhibitoren und EGFR Hemmer bezeichnet.

7. Pharmazeutische Zusammensetzung nach einem der vorgenannten Ansprüche, **dadurch gekennzeichnet, dass** sie in Form einer Trocken Pulverinhalationszusammensetzung oder einer druckbeaufschlagt gemessene Dosis Inhalator Zusammensetzung, vorzugsweise in Form einer Trocken Pulverinhalationszusammensetzung vorliegt.

8. Pharmazeutische Zusammensetzung nach Anspruch 8, zur Verwendung in einem Verfahren zur Behandlung von Erkrankungen der Atemwege, insbesondere Asthma und COPD.

## Revendications

1. Composition pharmaceutique en forme sèche appropriée pour l'administration par inhalation, **caractérisée en ce qu'**elle comporte un complexe de tiotropium ou son sel, ester, solvate, polymorphe, ou hydrate pharmaceutiquement acceptable et β - cyclodextrine ou leurs dérivés, ledit complexe possédant une taille particulaire moyenne allant de 1 à 10 µm, dans lequel les dérivés de β-cyclodextrine sont 2-hydroxypropyl-β-cyclodextrine, sulfobutylether-β-cyclodextrin, le sel de soude de β -cyclodextrin, et β-cyclodextrin ou β-cyclodextrin ramifié méthylé aléatoirement.

2. Composition pharmaceutique selon la revendication 1, **caractérisée en ce qu'**elle comprend de préférence le sel de bromure de tiotropium comme tiotropium ou son sel, ester, solvate, polymorphe, ou hydrate pharmaceutiquement acceptable.

3. Composition pharmaceutique selon la revendication 1, **caractérisée en ce que** le complexe est constitué de tiotropium ou son sel, ester, solvate, polymorphe, ou hydrate pharmaceutiquement acceptable et β-cyclodextrine ou leurs dérivés, et possédant une taille particulaire moyenne allant de 1 à 5 µm, et dans lequel les dérivés de β-cyclodextrine sont 2-hydroxypropyl-β-cyclodextrine, sulfobutylether-β-cyclodextrin, le sel de soude de β-cyclodextrin, et β-cyclodextrin ou β-cyclodextrin ramifié méthylé aléatoirement.

4. Composition pharmaceutique selon la revendication 1, **caractérisée en ce qu'**elle comprend le complexe constitué de tiotropium ou son sel, ester, solvate, polymorphe, ou hydrate pharmaceutiquement acceptable et β-cyclodextrine ou leurs dérivés, dans la proportion de 0,001% à 50%, dans lequel les dérivés de β-cyclodextrine sont 2-hydroxypropyl-β-cyclodextrine, sulfobutylether-β-cyclodextrin, le sel de soude de β -cyclodextrin, et β-cyclodextrin ou β-cyclodextrin ramifié méthylé aléatoirement.

5. Composition pharmaceutique selon la revendication 1, **caractérisée en ce qu'**elle comprend le complexe constitué de tiotropium ou son sel, ester, solvate, polymorphe, ou hydrate pharmaceutiquement acceptable et β-cyclodextrine ou leurs dérivés, dans la proportion de 0,01% à 10% en poids, dans lequel les dérivés de β-cyclodextrine sont 2- hydroxypropyl- β-cyclodextrine, sulfobutylether-β-cyclodextrin, le sel de soude de β-cyclodextrin, et β-cyclodextrin ou β-cyclodextrin ramifié méthylé aléatoirement.

6. Composition pharmaceutique selon la revendication 1, **caractérisée en ce qu'**elle comporte au moins un composant actif choisi parmi le group des autres principes actifs anticholinergiques, antagonistes adrénergiques, agents antiallergiques, agents anti-inflammatoires, antihistaminiques, stéroïdes, antagonistes des récepteurs de leukotriène, agents antimuscasrinique, inhibiteurs de PDE, et inhibiteurs d'EGFR pour l'administration séparément, séquentiellement, et simultanément.

7. Composition pharmaceutique selon l'une quelconque des revendications précédentes, **caractérisée en ce qu'**elle est en forme d'une composition de poudre sèche administrée par inhalation ou une composition administrée par inhalation de l'aérosol-doseur, de préférence en forme d'une composition de poudre sèche administrée par inhalation.

8. Une composition pharmaceutique selon la revendication 8 pour l'utilisation d'une méthode de traitement des maladies, particulièrement de l'asthme et la maladie pulmonaire obstructive chronique (MPOC).
